# EUROPEAN PATENT SPECIFICATION

(11) **EP 3 760 197 B1**
(45) Date of publication and mention of the grant of the patent: **27.04.2022**
(21) Application number: 19761332.6
(22) Date of filing: 02.02.2019
(51) Int. Cl.: A61K 31/136, A61K 31/198, A61P 35/00

(54) **TRANSPORT PROTEIN INHIBITOR FOR REDUCING EXPRESSION OF PD-1**
TRANSPORTPROTEININHIBITOR ZUR REDUKTION DER EXPRESSION VON PD-1
INHIBITEUR DE PROTÉINE DE TRANSPORT POUR RÉDUIRE L'EXPRESSION DE PD-1

(30) Priority: 02.03.2018 CN 201810176164
(43) Date of publication of application: 06.01.2021
(73) Proprietor: Institute of Basic Medical Sciences, Chinese Academy of Medical Sciences, Beijing 100005 (CN)
(72) Inventor: HUANG, Bo, Beijing 100005 (CN); LIU, Yuying, Beijing 100005 (CN)
(74) Representative: Heubeck, Christian
(86) International application number: PCT/CN2019/074548
(87) International publication number: WO 2019/165884

(56) References cited:
- CN-A- 103 547 923
- ALMASI SHEKOUFEH ET AL: "Exploring the Therapeutic Potential of Membrane Transport Proteins: Focus on Cancer and Chemoresistance", CANCERS, vol. 12, no. 6, 19 June 2020 (2020-06-19), page 1624, XP55775368, DOI: 10.3390/cancers12061624 Retrieved from the Internet: URL:https://www.ncbi.nlm.nih.gov/pmc/artic les/PMC7353007/pdf/cancers-12-01624.pdf>
- Yuying Liu,,Xiaoyu Liang, Wenqian Dong, Yi Fang, Jiadi Lv, Tianzhen Zhang, Roland Fiskesund, Jing Xie, Jinyan Liu, Xiaonan Yin,,Xu: "Tumor-Repopulating Cells Induce PD-1 Expression in CD8+ T Cells by Transferring Kynurenine and AhR Activation", Cancer Cell, vol. 33, no. 3, 12 March 2018 (2018-03-12) , pages 480-494, XP055634835, ISSN: 1535-6108, DOI: 10.1016/j.ccell.2018.02.005
- Chun Sung KIM, Seon-Ho CHO,Hong Sung CHUN,Sook-Young LEE, Hitoshi ENDOU,Yoshikatsu KANAI , Do Kyung KIM: "BCH, an Inhibitor of System L Amino Acid Transporters, Induces Apoptosis in Cancer Cells", Biol. Pharm. Bull., vol. 31, no. 6, 30 June 2008 (2008-06-30), pages 1096-10100, XP055634843, DOI: :10.1248/bpb.31.1096
- Jian Yang, Xu Hong-Xia: "Regulation of mTORC1 on Amino Acid Sensing and its Role in the Development of Cancer", Electronic Journal of Metabolism and Nutrition of Cancer, vol. 4, no. 2, 30 June 2017 (2017-06-30), pages 149-154, XP055729805, ISSN: 2095-7807, DOI: 10.16689/j.cnki.cn11-9349/r.2017.02.004
- Yangzom D. Bhutia, Ellappan Babu, Sabarish Ramachandran, ,Vadivel Ganapathy: "Amino Acid Transporters in Cancer and Their Relevan - ce to ''Glutamine Addiction '': Novel Targets for the Design of a New Class of Anticancer Drugs", Cancer Research, vol. 75, no. 9, 8 April 2015 (2015-04-08), pages 1782-1788, XP055634856, ISSN: 0008-5472, DOI: 10.1158/0008-5472.CAN-14-3745
- Morand Piert, Xia Shao, David Raffel, Mathew S. Davenport, Jeffrey Montgomery, Lakshmi Priya Kunju, Brian G. Hockley, Javed Siddiq: "Preclinical Evaluation of 11C-Sarcosine as a Substra- te of Proton-Coupled Amino Acid Transporters and First Human Application in Prostate Cancer", The Journal of Nuclear Medicine, vol. 58, no. 8, 31 August 2017 (2017-08-31), pages 1216-1223, XP055634864, ISSN: 0161-5505, DOI: 10.2967/jnumed.116.173179

## Description

### TECHNICAL FIELD

The present disclosure relates a transport protein inhibitor for use in preventing or treating cancer by reducing the expression of PD-1.

### BACKGROUND

Tumor remains one of the most fatal threats to human health. Solid tumors are responsible for most of those deaths. Although there have been significant advances in the medical treatment of certain tumors, the overall 5-year survival rate for all tumors has improved only about 10% over the past 20 years. Tumors or malignant tumors grow and metastasize rapidly in an uncontrolled manner, which makes timely detection and treatment extremely difficult.

Tumor immunotherapy is another tumor treatment method that is developed after surgery, radiotherapy and chemotherapy and has definite efficacy on treating tumor. Tumor immunotherapy resists and kills tumor cells by activating the immune system of one's own body, and is one of the development directions of tumor treatment in the future.

Chun Sung Kim et al, Biol. Pharm. Bull., vol 31, no.6 (2008), pp. 1096-10100 disclose that BCH can inhibit LAT1 activity, which leads to cancer cell apoptosis, wherein the process of cancer cell apoptosis is realized by means of inducing intracellular depletion of neutral amino acids necessary for cancer cell growth.

Different patients treated by different immunotherapies will exhibit different information, thus requiring specific immune system evaluation for patients treated by tumor immunotherapy to monitor the therapeutic effects of tumor immunotherapy.

The programmed death receptor-1 (PD-1) is a protein that has been linked to the suppression of the response of immune system during chronic infections, pregnancy, tissue allografts, autoimmune diseases and tumors. PD-L1 modulates immune responses by binding to an inhibitory receptor, which is referred to as programmed death receptor-1 (PD-1) and is expressed on the surface of T cells, B cells and monocytes. The mechanism of PD-1 immunotherapy is designing a specific protein antibody against PD-1 or PD-L1, preventing the recognition process between PD-1 and PD-L1, and partially restoring the function of tumor-specific cytotoxic CD8⁺ lymphocytes (CTLs) with antigenic specificity, so as to enable CTLs to kill tumor cells. However, despite the unprecedented success of the clinical application of programmed cell death receptor-1 (PD-1) antibodies against many types of tumors, the underlying mechanism of how PD-1 is regulated in tumor-specific cytotoxic CD8⁺ lymphocytes (CTLs) in tumor microenvironment remains poorly characterized. Meanwhile, considering the relationship between tumor relapse and the remaining tumorigenic cells, it is possible that CTLs, even after being relieved from the inhibitory effect of PD-1 signaling, may be incapable of destroying stem cell-like cancer cells that are able to repopulate tumors [tumor-repopulating cells (TRCs)]. These TRCs are a self-renewing and highly tumorigenic subpopulation of cancer cells that play crucial roles in the initiation, promotion and progression of tumorigenesis.

Furthermore, there still remains relapse in part of the treated patient population who initially respond and show extraordinary treatment outcomes. Therefore, medical personnel still need a novel regimen for treating tumor diseases.

### SUMMARY

The present disclosure provides a transport protein inhibitor selected from inhibitors of SLC1A5, SLC7A8 and PAT4 for use in preventing or treating cancer by reducing the expression of PD-1, wherein the patient has been identified as having cancer accompanied with increased PD-1 expression, and wherein the transport protein inhibitor is selected from one or more of L-γ-glutamyl-p-nitroanilide (GPNA), sarcosine (SAR), 2-amino-2-norboranecarboxylic acid (BCH), and pharmacologically acceptable salts thereof.

In the present disclosure, the cancer accompanied with increased PD-1 expression is preferably selected from one or more of melanoma accompanied with increased PD-1 expression, liver cancer accompanied with increased PD-1 expression, breast cancer accompanied with increased PD-1 expression, lung cancer accompanied with increased PD-1 expression, colorectal cancer accompanied with increased PD-1 expression, ovarian cancer accompanied with increased PD-1 expression, or leukemia accompanied with increased PD-1 expression.

In the present disclosure, the above transport protein inhibitor may be combined with other agent(s) for treating the cancer accompanied with increased PD-1 expression; preferably, said other agent is selected from one or more of an immunomodulator, an IDO inhibitor, an AhR inhibitor, a radiotherapeutic agent or a chemotherapeutic agent; alternatively, the immunomodulator is selected from an immune checkpoint modulator or an agent that facilitates or mediates an antigen presentation that promotes a cell-mediated immune response.

In the present disclosure, treating or preventing the cancer accompanied with increased PD-1 expression may further comprise a step of surgical treatment.

### BRIEF DESCRIPTION OF THE DRAWINGS

Fig. 1 shows that IFN-γ from CTLs stimulates TRCs to upregulate PD-1 expression in CTLs.
Fig. 2 shows that Kyn upregulates PD-1 expression in CD8⁺ T cells.
Fig. 3 shows that Kyn activates aryl hydrocarbon receptor (AhR) for upregulation of PD-1.
Fig. 4 shows that AhR upregulates the expression of SLC7A8 and PAT4 to promote the transport of Kyn.
Fig. 5 shows that TCR signaling promotes the upregulaton of PD-1 by Kyn.
Fig. 6 shows that Kyn upregulates PD-1 expression *in vivo* by activating AhR.
Fig. 7 shows that PD-1 expression is regulated by *in-vivo* Kyn-AhR pathway in patients.

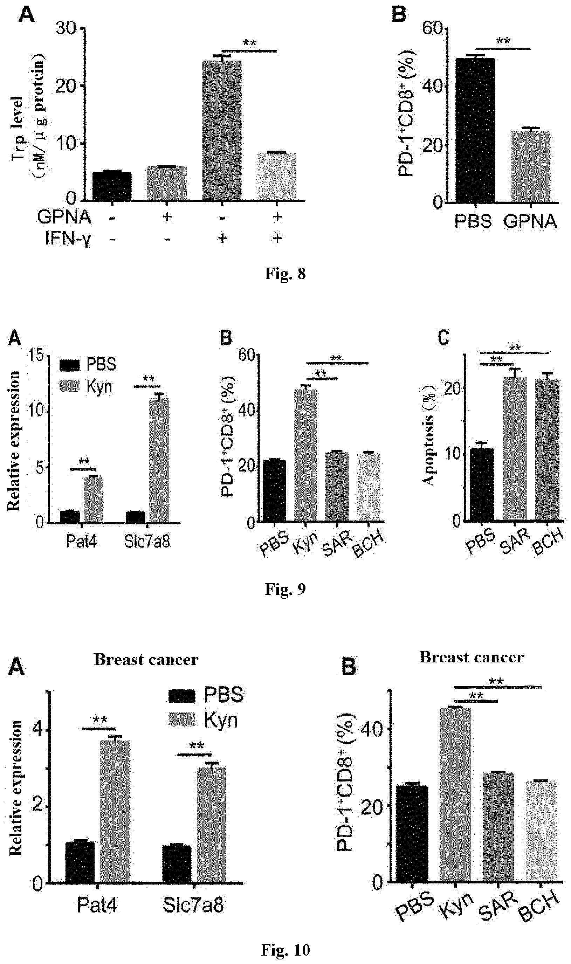

Fig. 8 shows the determination results of the effect of inhibiting the tryptophan transporter SLC1A5 in tumor cells on the expression of PD-1 in specific CD8⁺ T cells. Among them, Fig. 8A shows the uptake of tryptophan by tumor cells which is determined in a case where IFN-γ is added into OVA-B16 melanoma cell culture system and GPNA as an inhibitor of the tryptophan transporter SLC1A5 is added; and Fig. 8B shows the determination results of the effect of GPNA (an SLC1A5 inhibitor) on the expression of PD-1 in CD8⁺ T cells in a case where 3D-cultured OVA-B16 cells are co-cultured with specific OT-1 CD8⁺ T cells and GPNA (an SLC1A5 inhibitor) is added to the culture system.
Fig. 9 shows the determination results of the effect of inhibiting Kyn transporters PAT4 and SLC7A8 in mouse CD8⁺ T cells on the expression of PD-1 in CD8⁺ T cells. Among them, Fig. 9A shows the determination results of the expression of the transporters PAT4 and SLC7A8 in a case where Kyn is added to the activated OT-1 CD8⁺ T cells culture system; Fig. 9B shows the determination results of the effects of SAR and BCH on the expression of PD-1 in CD8⁺ T cells in a case where Kyn is added to OT-1 CD8⁺ T cells that are cultured and activated *in vitro* while SAR or BCH (inhibitor of PAT4 or SLC7A8) is added thereto at the same time; and Fig. 9C shows the determination results of the killing effect of specific T cells on tumor cells in a case where 3D-cultured OVA-B16 cells are co-cultured with specific OT-1 CD8⁺ T cells and SAR or BCH is added to the culture system.
Fig. 10 shows the determination results of the effect of inhibiting Kyn transporters PAT4 and SLC7A8 in human peripheral blood CD8⁺ T cells on the expression of PD-1 in CD8⁺ T cells. Among them, Fig. 10A shows the determination results of the expression of the transporters PAT4 and SLC7A8 in a case where Kyn is added to the culture system of the activated human peripheral blood CD8⁺ T cells; and Fig. 10B shows the determination results of the effects of SAR and BCH on the expression of PD-1 in CD8⁺ T cells in a case where Kyn is added to the activated human peripheral blood CD8⁺ T cells while SAR or BCH (inhibitor of PAT4 or SLC7A8) is added thereto at the same time.

### DETAILED DESCRIPTION

### Definitions

When used in combination with the term "comprise" in claims and/or specification, the wording "a" or "an" may refer to "one", but may also refer to "one or more", "at least one" and "one or more than one".

As used in claims and specification, the wording "comprise", "have", "include" or "contain" means inclusive or open-ended, and does not exclude additional and unmentioned elements, method or steps.

Throughout the application document, the term "about" means that a value includes the standard deviation of the error of the device or method used to determine the value.

Although the definition of the term "or" as being an alternative only and as "and/or" are both supported by the disclosed content, the term "or" in claims means "and/or" unless it is explicitly indicated that the term "or" only means an alternative or the alternatives are mutually exclusive.

The term "transport protein" or "transporter" refers to a carrier protein on the cell membrane of a tissue. In the present disclosure, the above terms may be used interchangeably and have the same meaning.

The definition of the term "drug" is a substance capable of preventing or treating a disease. A drug may comprise one effective ingredient for treating disease, and may also comprise two or more effective ingredients for treating disease. Alternatively, said drug may be a vaccine. Said "drug" may also be added with ingredients as needed, for example, ingredients that facilitate extended storage period of drug, stability or better absorption by the patient. Illustratively, said ingredients may be selected from pharmaceutical excipients.

The term "immunomodulator" comprises an immune checkpoint modulator. For example, immune checkpoint protein receptors and their ligands mediate the suppression of T cell-mediated cytotoxicity and are often expressed by tumors or expressed on anergic T cells in the tumor microenvironment, thus permitting the tumor to evade immune attack. Inhibitors of the activity of immunosuppressive checkpoint protein receptors and their ligands may overcome the immunosuppressive tumor environment, so as to permit cytotoxic T cell attack on tumor. Examples of immune checkpoint proteins include but are not limited to PD-1, PD-L1, PDL2, CTLA4, LAG3, TIM3, TIGIT and CD103. Modulation (including inhibition) of the activity of such protein may be accomplished by an immune checkpoint modulator, which may include, for example, an antibody, an aptamer, a small molecule, a soluble form of a checkpoint receptor protein and the like that target a checkpoint protein. PD-1-targeting inhibitors include the approved drug agents pembrolizumab and nivolumab, while ipilimumab is an approved CTLA-4 inhibitor. Antibodies specific for PD-L1, PD-L2, LAG3, TIM3, TIGIT and CD103 are known and/or commercially available, and may also be produced by those skilled in the art.

In addition to immune checkpoint modulators, immunomodulators also include an agent that facilitates or mediates an antigen presentation that promotes a cell-mediated immune response. Such immunomodulators may include, for example, a tumor antigen vaccine. A tumor antigen vaccine may include a preparation comprising a particular tumor antigen or a set of known tumor antigens, with or without a subject's own dendritic cells or an adjuvant. Or, a tumor antigen vaccine may comprise a relatively crude preparation of a tumor cell antigen derived from a patient's tumor. When said tumor cell antigen is exposed *ex vivo* to the dendritic cells generated *in vitro* from a patient's cells, T cell-mediated attack on tumor is allowed when the dendritic cell vaccine is introduced into the patient. In one embodiment, immunomodulators include a tumor antigen vaccine. In another embodiment, the tumor antigen vaccine comprises a dendritic cell tumor antigen vaccine.

The term "IDO" (indoleamine 2,3-dioxygenase) refers to a protease involved in the metabolism of tryptophan. Indoleamine 2,3-dioxygenase 1 (IDOl) modulates immune cell function to a suppressive phenotype, and is therefore partially accountable for tumor escape from host immune surveillance. IDO1 degrades the essential amino acid tryptophan into kynurenine and other metabolites. These metabolites and the lack of tryptophan lead to the suppression of the function of effector T cells and enhanced differentiation of regulatory T cells. IDO inhibitors are known and/or commercially available, and may also be produced by those skilled in the art.

The term "kynurenine (Kyn)" refers to an intermediate metabolite of tryptophan generated in human body.

The term "AhR (aryl hydrocarbon receptor) inhibitor" or "AhR antagonist" refers to such an agent or compound that inhibits one or more AhR signaling and downstream effector pathways, including constitutive AhR signaling. Therefore, the term "AhR inhibitor" refers to such an agent that inhibits the expression of AhR polypeptide or the polynucleotide encoding AhR, or binds to partially or completely block the stimulating effect of said AhR polypeptide or the polynucleotide encoding AhR, reduces, prevents and delays the activating effect of said AhR polypeptide or the polynucleotide, and inactivates, desensitizes or downregulates the activity of said AhR polypeptide or the polynucleotide encoding AhR. Such AhR inhibitors are capable of, for example, inhibiting AhR expression (for example, AhR translation, the post-translational processing of AhR, the stabilization, degradation, nuclear localization or cytoplasmic localization of AhR polypeptide, or the transcription, post-transcriptional processing, stabilization or degradation of the polynucleotide encoding AhR), or binding to partially or completely block the stimulating effect, DNA binding, or the transcription factor activity of AhR. AhR inhibitors are capable of taking effect directly or indirectly. AhR inhibitors are known and/or commercially available, and may also be produced by those skilled in the art.

The term "radiotherapeutic agent" includes drugs that cause DNA damage. Radiotherapy has been widely used in the treatment of cancer and diseases, and includes those commonly referred to as γ-ray and X-ray and/or targeted delivery of radioisotopes to tumor cells.

The term "chemotherapeutic agent" refers to a chemical compound useful for treating cancer. Classes of chemotherapeutic agents include but are not limited to: an alkylating agent, an antimetabolite, a kinase inhibitor, a spindle poison plant alkaloid, a cytotoxic/antitumor antibiotic, a topoisomerase inhibitor, a photosensitizer, an anti-estrogen, a selective estrogen receptor modulator, an anti-progesterone, an estrogen receptor downregulator, an estrogen receptor antagonist, a luteinizing hormone-releasing hormone agronist, anti-androgens, an aromatase inhibitor, an EGFR inhibitor, a VEGF inhibitor, an antisense oligonucleotide that inhibits the expression of gene(s) involved in abnormal cell proliferation or tumor growth. Chemotherapeutic agents that may be used in the treatment method of the present disclosure include a cell growth inhibitor and/or a cytotoxic agent.

The term "pharmaceutical excipient" refers to a substance that is a basic material and an important constituent part of a pharmaceutical preparation. A pharmaceutical excipient not only endows a drug with a certain dosage form, but is also closely related to the improvement of drug efficacy and the reduction of adverse reaction. In addition to endowing with a dosage form, serving as a carrier and enhancing the stability, pharmaceutical excipients also have important functions such as solubilizing, aiding solubilization and extending or controlling the release, and are important ingredients that may influence the quality, safety and effectiveness of drugs. Said pharmaceutical excipients may be classified into various types, such as a solvent, a propellant, a solubilizer, a cosolvent, an emulsifier, a colorant, a binder, a disintegrant, a filler, a lubricant, a wetting agent, an osmotic pressure regulator, a stabilizer, a glidant, a flavoring agent, a preservative, a suspending agent, a coating material, an aromatic agent, an anti-adhesive agent, an integrating agent, a penetration enhancer, a pH regulator, a buffer, a plasticizer, a surfactant, a foaming agent, a defoaming agent, a thickening agent, an inclusion agent, a humectant, an absorbent, a diluent, a flocculant, a deflocculant, a filter aid, and a release retarder.

### Examples

Other objects, features and advantages of the present disclosure will become apparent from the following detailed description. However, it should be understood that, the detailed description and specific examples (although representing the specific embodiments of the present disclosure) are given for illustrative purpose only.

The experimental animals, reagents and materials used in the present disclosure were as follows.

Female C57BL/6 mice (6- to 8-weeks old), OT-1 transgenic mice, Pmel-1 transgenic mice, AhR-/- mice and MMTV-PyMT mice were all purchased from commercial institutions. These animals were maintained in the corresponding facilities owned by the applicant under pathogen-free conditions. All studies involving mice were approved by the Animal Care and Use Committee.

Mouse tumor cell lines OVA-B16 (melanoma) , H22 (hepatocellular carcinoma) and ID8 (ovarian cancer) as well as human tumor cell lines A375 (melanoma) and HepG2 (hepatocellular carcinoma) were purchased from China Center for Type Culture Collection (Beijing, China) and cultured in DMEM (Thermo Scientific) with 10% fetal bovine serum (FBS) (Gibco, USA), except that H22 cells were grown in RPMI 1640 culture medium (Gibco, USA) containing 10% FBS.

pGFP-C-shLenti-shIDO1, pGFP-C-shLenti-shSLC1A5, pGFP-V-RS-shPAT4, pGFP-V-RS-shSLC7A8 and pCMV6-Entry-DDK-SLC1A5 were purchased from Origene (MD, USA). Kynurenine, kynurenic acid, indoxyl sulfate, melatonin,1-L-MT, 3',4'-dimethoxyflavone (DMF), TCDD, sarcosine (SAR), 2-amino-2-norbornanecarboxylic acid (BCH), L-γ-Glutamyl-p-nitroanilide (GPNA) and anti-PAT4 antibody were from Sigma-Aldrich (ST, USA). Cyclosporine A was from SelleckChemicals. Anti-IFN-y, TNF-α, PD-1 and PD-L1 neutralizing antibody as well as anti-PD-1, CD3, CD8, IFN-γ, TNF-α and CD107a antibodies were purchased from BioLegend. IFN-γ was purchased from R&D Systems. Salmon fibrinogen, thrombin and dispase were from Reagent Proteins (CA, USA). Puromycin was from Invitrogen (SD, USA).

Human peripheral blood and resected human breast or colon cancer tissues were obtained from patients at hospitals. Ethical permission was granted by the Clinical Trial Ethics Committee of the corresponding hospital. All patients provided written informed consent to participate in this study.

### Experimental methods

### (1) Detection methods of biomarkers

In any of the aforementioned methods, the presence and/or expression level/amount of a biomarker was determined by measuring the protein expression level of the biomarker. In certain embodiments, said method comprised bringing a biological sample into contact with an antibody that specifically binds to the biomarker described herein (for example, an anti-PD-1 antibody) under a condition where the binding of the biomarker was allowed, and detecting whether a complex was formed by the antibody and the biomarker. Such method may be an *in-vitro* or *in-vivo* method. In some cases, an antibody such as a biomarker used for selecting individuals was used to select subjects eligible for adopting PD-1 axis binding antagonist therapy. Any method for measuring the protein expression level that was known in the art or provided herein could be used. For example, in some embodiments, the protein expression level of a biomarker (for example, PD-1) was determined by a method selected from the group consisting of the following methods: flow cytometry (for example, fluorescence-activated cell sorting (FACSTM)), Western blotting, enzyme-linked immunosorbent assay (ELISA), immunoprecipitation, immunohistochemistry (IHC), immunofluorescence, radioimmunoassay, dot blotting, immunoassay, surface plasmon resonance, spectroscopy, mass spectrometry, and HPLC.

### (2) Tumor cells cultured on 2D rigid dish or in 3D fibrin gels

For conventional 2D cell culture, tumor cells were maintained in a rigid dish with complete culture medium. Specifically, fibrinogen (Searun Holdings Company, Freeport, ME) was diluted to 2 mg/ml with T7 buffer (pH 7.4, 50 mM Tris, 150 mM NaCl). Then, a 1:1 (volume) mixture of fibrinogen and cell solution was made. 250 µl of the cell/fibrinogen mixture was seeded into a 24-well plate and mixed well with 5 µl pre-added thrombin (0.1 U/µl, Searun Holdings Company). After 30 min of incubation at 37°C, these cells were supplemented with 1 ml of complete culture medium. After 5 days of incubation, dispase II was added to and digested the cultured gels for 10 min at 37°C. The spheroids were harvested and further digested with 0.25% trypsin for 3 min to obtain a single cell suspension for the following experiments.

### (3) Statistical analysis methods

All experiments were performed at least three times. Results were expressed as mean ± SEM and analyzed by Student's t-test. P value < 0.05 was considered statistically significant. The analysis was conducted using the Graphpad 6.0 software. Sample exclusion was never carried out.

### Example 1: PD-1 expression in tumor-specific CD8⁺ T cells was induced by IFN-γ-stimulated TRCs

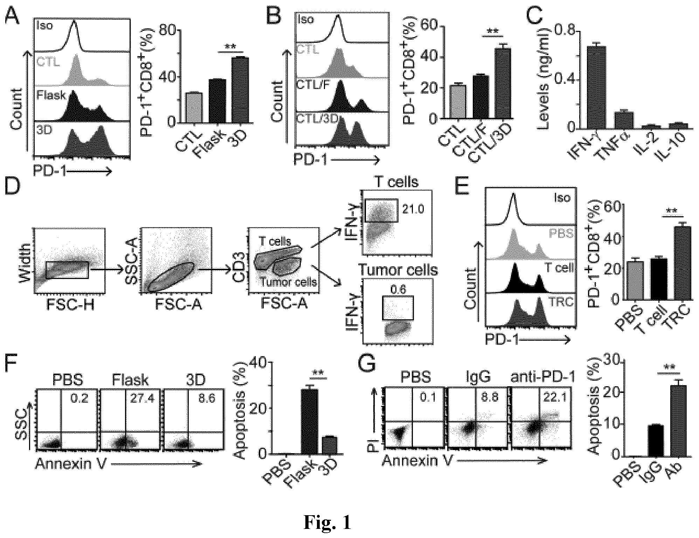

The steps of the technical solution adopted in this example and the corresponding results were as follows.
(1) Anti-CD3/CD28 bead-activated OVA-CTLs were cultured alone or together with OVA-B16 cells (differentiated OVA-B16 cells) from conventional culture flasks or OVA-B16 TRCs grown in 3D fibrin matrices for 24 hours. PD-1 expression was determined by flow cytometry. The determination results were as shown in Fig. 1A.
(2) The co-culture supernatants of CTLs with either differentiated OVA-B16 cells or OVA-B16 TRCs or unmodified culture medium (negative control) were concentrated by freeze-drying and incubated with anti-CD3/CD28 bead-activated OVA-CTLs for 24 hours. PD-1 expression was determined by flow cytometry. The determination results were as shown in Fig. 1B.
(3) CD3/CD28 bead-activated OVA-CTLs were cultured for 24 hours and the supernatant was analyzed by ELISA. The determination results were as shown in Fig. 1C.
(4) Anti-CD3/CD28 bead-activated OVA-CTLs were co-cultured with OVA-B16 TRCs for 24 h. IFN-γ production in T cells and tumor cells was analyzed by flow cytometry. The determination results were as shown in Fig. 1D.
(5) Anti-CD3/CD28 bead-activated OVA-CTLs were treated with PBS (phosphate buffered saline) or the conditioned medium from CTLs or OVA-B16 TRCs stimulated by IFN-γ for 24 h. PD-1 expression of OVA-CTLs was subsequently determined by flow cytometry. The determination results were as shown in Fig. 1E.
(6) OVA-B16 cells from conventional culture flasks or OVA-B16 TRCs from 3D fibrin gels were co-cultured with anti-CD3/CD28 bead-activated OVA-CTLs at a ratio of 1:30 for 4 h. CD45⁻ tumor cell apoptosis was determined by flow cytometry. The determination results were as shown in Fig. 1F.
(7) OVA-B16 TRCs were co-cultured with anti-CD3/CD28 bead-activated OVA-CTLs at a ratio of 1:30 in the presence of either a neutralizing anti-PD-1 antibody or an IgG isotype control. After 4 h, cell viability was analyzed by flow cytometry. The determination results were as shown in Fig. 1G.

As could be seen from the results of Example 1, by using B16 melanoma TRCs expressing the model tumor antigen ovalbumin (OVA) and OVA-specific CTLs derived from OT-1 T-cell receptor transgenic mice, the inventors found that TRCs strongly induced anti-CD3/CD28 bead-activated OT-1 T cells to upregulate PD-1 expression during co-incubation (Fig. 1A). Such PD-1 upregulation was also confirmed in gp100-specific CD8⁺ T cells as well as in OVA peptide-activated OT-1 cells. Notably, the above increase of PD-1⁺ cells was ascribed to the conversion of PD-1⁻ cells to PD-1⁺ T cells, but not due to the depletion of PD-1⁻ cells or the expansion of PD-1⁺cells, based on that co-incubation of PD-1⁻ OT-1 cells with OVA-B16 TRCs, where PD-1^{÷} T cells appeared, and co-incubation of PD-1⁺ cells with or without TRCs, where the proliferation of PD-1⁺ T cells was not altered while PD-1 expression was upregulated. Furthermore, the above co-culture supernatants were capable of upregulating PD-1 expression in unrelated CD8⁺ T cells (Fig. 1B), suggesting that TRCs or T cells themselves released certain factor(s) to induce PD-1 expression. To identify the factor(s) produced by interacting cells that augment(s) PD-1 expression in T cells, the inventors found that the supernatants from TRC monocultures were unable to elevate PD-1 expression, indicating that T cells were required for the production of the soluble factor(s) that upregulated PD-1. Activated T cells released a multitude of cytokines into the supernatant, most notably IFN-γ (Fig. 1C). Remarkably, the inventors found that IFN-γ neutralization impeded the above described PD-1 upregulation, whereas TNF-α neutralization did not. In line with this, IFN-γ was found to be only expressed by OT-1 cells but not by TRCs (Fig. 1D). Having identified IFN-γ as a key molecule, the inventors treated T cells and TRCs individually with recombinant IFN-γ, and found that only supernatants from IFN-γ-treated TRCs were able to upregulate PD-1 expression in T cells (Fig. 1E). Therefore, these data suggested that during the interaction between TRCs and tumor-infiltrating T cells, IFN-γ produced by T cells directly stimulated TRCs to release factor(s) that promoted PD-1 expression. Here, the inventors also confirmed that this upregulated PD-1 was functional. The inventors found that OVA-specific CTLs were weakly cytotoxic against OVA-TRCs in the above co-incubation (Fig. 1F), however, adding a neutralizing anti-PD-1 antibody could markedly improve the cytotoxicity of CTLs to TRCs (Fig. 1G). The effect of the blocking antibody was not driven by upregulation of PD-L1 by T cell-derived factors on TRCs. Indeed, the expression of PD-L1 was upregulated by IFN-γ; however, an additional supplement of anti-PD-L1 antibody to the co-culture had no effect on TRC apoptosis.

### Example 2: Kynurenine produced by TRCs was identified to mediate the upregulation of PD-1, and IFN-γ upregulated the tryptophan transporter SLC1A5 in TRCs to facilitate Trp uptake and Kyn production

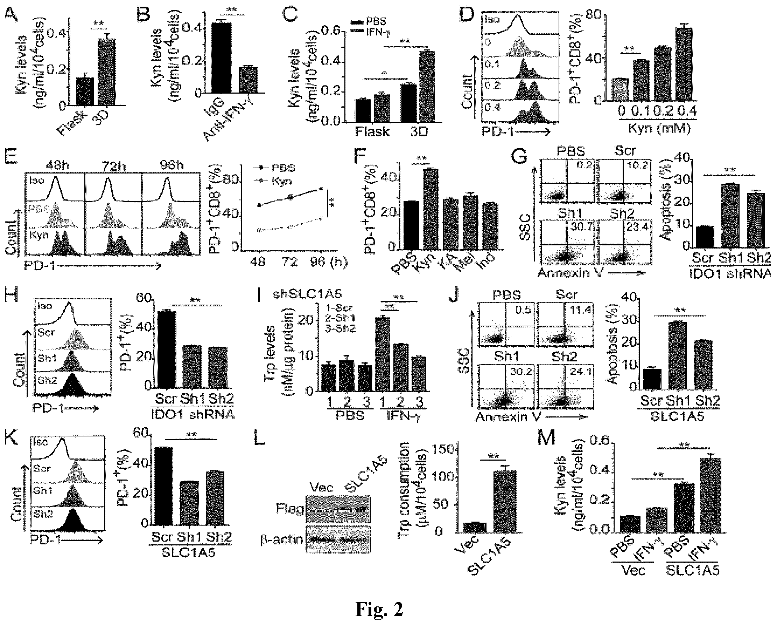

The steps of the technical solution adopted in this example and the corresponding results were as follows.
(1) Anti-CD3/CD28 bead-activated OVA-CTLs were co-cultured with OVA-B16 cells from conventional culture flasks or OVA-B16 TRCs grown in 3D fibrin matrices for 24 hours. Kyn in the supernatant was determined by ELISA. The determination results were as shown in Fig. 2A.
(2) CD3/CD28 bead-activated OVA-CTLs were co-cultured with OVA-B16 TRCs for 24 hours in the presence of a neutralizing anti-IFN-y antibody or an isotype control. Kyn levels in the supernatants were determined by ELISA. The determination results were as shown in Fig. 2B.
(3) OVA-B16 cells grown in culture flask or OVA-B16 TRCs were treated with IFN-γ (10 ng/ml) for 24 h. Kyn levels in the supernatants were determined by ELISA. The determination results were as shown in Fig. 2C.
(4) The isolated splenic CD8⁺ T cells were incubated with anti-CD3/CD28 beads in the presence of different concentrations of Kyn. PD-1 expression was determined by flow cytometry. The determination results were as shown in Fig. 2D.
(5) The experimental steps were the same as those of step (4), except that CD8⁺ T cells were treated with 200 µM Kyn for different time periods as shown in Fig. 2E. The determination results were as shown in Fig. 2E.
(6) Anti-CD3/CD28 bead-activated CTLs were treated with PBS, 200 µM Kyn or other metabolites (500 µM) from the tryptophan pathway (melatonin, kynurinic acid and indoxyle sulphate). After 24 h, PD-1 expression was determined by flow cytometry. The determination results were as shown in Fig. 2F.
(7) Scramble- or shIDO1-(Sh1 or Sh2)-OVA B16 TRCs were co-cultured with anti-CD3/CD28 bead-activated OT-1 T cells. After 4 h or 24 h, TRC apoptosis (as shown in Fig. 2G) or PD-1 expression on T cells (as shown in Fig. 2H) was analyzed by flow cytometry. The analysis results were as shown in Fig. 2G and Fig. 2H, respectively.
(8) Tryptophan levels in cell lysates were determined by ELISA in scramble- and shSLC1A5-(Sh1 or Sh2)-B16 TRCs in the presence or absence of IFN-γ for 24 h. The determination results were as shown in Fig. 21.
(9) Scramble- and shSLC1A5-(Sh1 or Sh2)-OVA B16 TRCs were co-cultured with anti-CD3/CD28 bead-activated OT-1 T cells. After 4 h or 24 h, TRC apoptosis (as shown in Fig. 2J) or PD-1 expression (as shown in Fig. 2K) was analyzed by flow cytometry. The analysis results were as shown in Fig. 2J and Fig. 2K, respectively.
(10) B16 TRCs were transiently transfected with vector or Flag-SLC1A5 plasmid. The over-expression of SLC1A5 was confirmed by western blot (left). Tryptophan levels in the supernatants were determined by ELISA and the Trp consumption was calculated (right). The confirmation results were as shown in Fig. 2L.
(11) Vec- or SLC1A5-B16 cells cultured in rigid plate were treated with PBS or IFN-γ (10 ng/ml) for 24 h. Kyn levels in the supernatants were determined by ELISA. The determination results were as shown in Fig. 2M. ^{∗}p<0.05, ^{∗∗}p<0.01. The data represented mean ± SEM.

As could be seen from the results of Example 2, much higher levels of Kyn were found in the supernatants of B16 TRC-T cell co-culture compared to the supernatants of differentiated B16 cell-T cell co-culture (Fig. 2A), and IFN-γ neutralization inhibited the production of Kyn (Fig. 2B). Consistent with this result, the inventors found that IFN-γ was able to directly stimulate B16 TRCs rather than differentiated B16 cells to release high levels of Kyn (Fig. 2C). Furthermore, TRCs generated from other tumor types (murine H22 hepatocarcinoma and human A375 melanoma) also released significant amount of Kyn upon IFN-γ stimulation. In order to verify that OVA-B16 TRCs used Kyn to upregulate PD-1 expression in CD8⁺ T cells, the inventors added Kyn directly to CD8⁺ T cells in the presence of anti-CD3/CD28 microbeads. The results showed that T cells markedly upregulated PD-1 expression in a dose and time-dependent manner (Figs. 2D and 2E). The inventors also found that Kyn-increased PD-1⁺ cells were ascribed to the conversion of PD-1⁻ cells to PD-1⁺ T cells, but not due to the depletion of PD-1⁻ cells or the expansion of PD-1⁺ cells. In addition to Kyn, other tryptophan catabolites (kynurenic acid, indoxyl sulfate and melatonin) were also tested for their ability to upregulate PD-1 expression. However, the inventors found that none of them was able to alter PD-1 expression on T cells (Fig. 2F). Here, the inventors also used tryptophan-free medium to co-culture OT-1 cells and TRCs as comparable approach to further investigate PD-1 upregulation by Kyn. The inventors found that tryptophan deficiency resulted in PD-1 downregulation concomitant with the upregulation of the expression of IFN-γ, TNF-α and CD107a (a degranulation marker for perforin/granzyme B) in T cells as well as enhancement of TRC apoptosis. In addition, the supernatant from the tryptophan-free co-culture was not able to upregulate PD-1 expression in anti-CD3/CD28-activated OT-1 T cells. Together, these data suggested that Kyn released by IFN-γ-stimulated TRCs was capable of upregulating PD-1 expression in T cells.

Meanwhile, basal IDO1 expression was much higher in all types of TRCs relative to their differentiated counterparts, and it could be further upregulated by IFN-γ. Also, when co-cultured with activated OT-1 T cells, IDO1 expression was upregulated in OVA-B16 TRCs, but TDO was not detectable. On the other hand, blocking IDO1 activity by the inhibitor 1-MT or knocking down IDO1 in OVA-B16 TRCs resulted in more TRC apoptosis in the co-culture system (Fig. 2G), as well as the downregulation of PD-1 (Fig. 2H) but the upregulation of the expression of IFN-γ, TNF-α and CD107a, suggesting that IDO1-Kyn specifically regulated PD-1 expression in CD8⁺ T cells. Although the induction of IDO1 expression was clearly critical for the initial phase of rapid Kyn production, maintaining a high Kyn production in TRCs required the import of enough extracellular tryptophan as the substrate of IDOl, especially considering that Kyn could be further metabolized and released into the extracellular space. Despite high consumption of tryptophan in TRCs due to high IDO1 activity, cytosolic tryptophan levels were not reduced in TRCs relative to differentiated tumor cells, suggesting that TRCs replenished their tryptophan storage by active transportation. Therefore, the inventors went on to investigate the expression of three common neutral amino acid transporters (SLC7A5, SLC7A8 and SLC1A5) that transported the essential amino acids into cells. The inventors found that TRCs had a higher expression of SLC1A5 but not SLC7A5 or SLC7A8, and that the expression of SLC1A5 was further augmented by IFN-γ treatment at both mRNA and protein levels. Moreover, knocking down SLC1A5 by shRNAs or blocking SLC1A5 activity by the inhibitor L-γ-Glutamyl-p-nitroanilide (GPNA) resulted in decreased intracellular levels of tryptophan in TRCs (Fig. 21), concomitant with more TRC apoptosis as well as PD-1 downregulation and the upregulation of the expression of IFN-γ, TNF-α and CD107a (Figs. 2J and 2K). Conversely, the overexpression of SLC1A5 increased tryptophan consumption (Fig. 2L) and boosted Kyn production in differentiated cells (Fig. 2M). Together, these data suggested that IFN-γ-upregulated SLC1A5 increased the uptake of tryptophan and thus enables TRCs to sustain an active tryptophan-IDO1-Kyn metabolic pathway.

### Example 3: Kyn activated aryl hydrocarbon receptor (AhR) for PD-1 upregulation in T cells

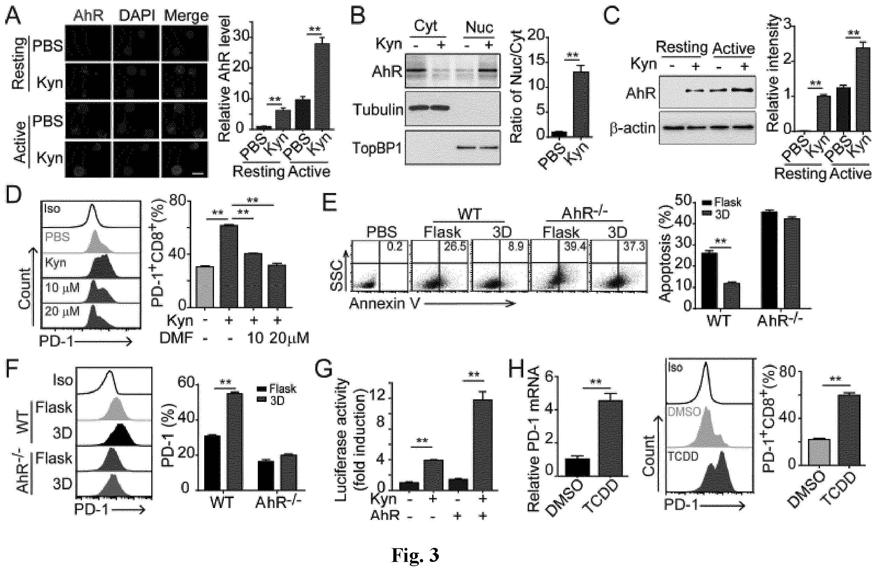

The steps of the technical solution adopted in this example and the corresponding results were as follows.
(1) CD8⁺ T cells isolated from spleen were stimulated with 200 µM Kyn or Kyn + anti-CD3/CD28 beads for 48 h. CD8⁺ T cells were fixed, stained with an anti-AhR antibody and imaged by confocal microscopy. The imaging results were as shown in Fig. 3A. Bar, 10 µM.
(2) The experimental steps were the same as those of step (1), except that the cytoplasmic and nuclear protein fractions were seperated prior to the western blot analysis of AhR. The intensity relative to that in the resting/Kyn group was calculated. The calculation results were as shown in Fig. 3B.
(3) Western blot analysis of AhR expression in both resting CD8⁺ T cells and anti-CD3/CD28 bead-activated CD8⁺ T cells treated with PBS or Kyn (200 µM) for 48 h was carried out. AhR ratio of Nuc/Cyt was calculated relative to that in the Kyn (-) group. The analysis results were as shown in Fig. 3C.
(4) CD8⁺ T cells activated by anti-CD3/CD28 beads were treated with PBS, Kyn or Kyn/DMF (10 or 20 µM) for 72 h. The expression of PD-1 was determined by flow cytometry. The determination results were as shown in Fig. 3D.
(5) Anti-CD3/CD28 bead-activated wild-type or AhR^{-/-} OT-1 mice were co-cultured with OVA-B16 cells or OVA-B16 TRCs. Tumor cell apoptosis (as shown in Fig. 3E) or PD-1 expression in T cells (as shown in Fig. 3F) was analyzed by flow cytometry. The analysis results were as shown in Fig. 3E and Fig.3F, respectively.
(6) HEK293T cells were transiently co-transfected with the luciferase reporter PGL3 coupled to the PD-1 promoter together with an AhR or an empty plasmid for 24 h and then treated with Kyn for another 48 h. The test results were as shown in Fig. 3G.
(7) Activated CD8⁺ T cells were treated with TCDD for 48 h and then PD-1 expression was analyzed by qPCR (left) and flow cytometry (right). The analysis results were as shown in Fig. 3H. ^{∗∗} p<0.01. The data represented mean ± SEM.

As could be seen from the results of Example 3, the inventors found that the addition of Kyn resulted in the translocation of cytoplasmic AhR to the nucleus of activated T cells, as demonstrated by fluorescent microscopy and western blot (Figs. 3A and 3B). Intriguingly, in addition to AhR activation, the inventors also found that AhR, although lowly expressed in naive and activated T cells, was upregulated in resting T cells upon Kyn stimulation, which was further increased in activated T cells (Fig. 3C). On the other hand, inhibition of AhR activity using DMF or CH-223191 thwarted the Kyn-dependent upsurge of PD-1 (Fig. 3D). In order to further specifically confirm the role of AhR in PD-1 upregulation, the inventors used AhR^{-/-} OT-1 T cells to co-culture with OVA-B16 TRCs. Indeed, AhR deficiency resulted in more TRC apoptosis (Fig. 3E), concomitant with PD-1 downregulation (Fig. 3F). Moreover, when the inventors co-transfected HEK293T cells with a PD-1 luciferase reporter and a plasmid driving the overexpression of AhR, the inventors found that the addition of Kyn resulted in a 4-fold increase of the luciferase activity in HEK293T cells with intrinsic AhR expression (transfected with an empty control plasmid) and a 12-fold increase in cells that overexpressed AhR (Fig. 3G). Since AhR recognized various exogenous toxins for their function exertion, the inventors also tested TCDD, i.e., an exogenous ligand for AhR. Consistent with the hypothesis of the inventors, TCDD also upregulated PD-1 expression in T cells (Fig. 3H). Together, these data suggested that Kyn could upregulate PD-1 expression in CD8⁺ T cells through the induction and activation of AhR.

### Example 4: AhR transcriptionally regulated the expression of transporters for CD8⁺ T cells to take up exogenous Kyn

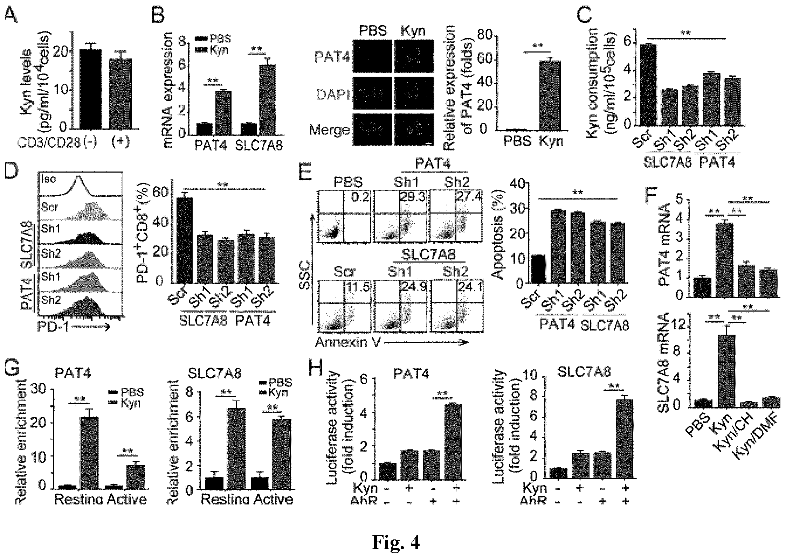

The steps of the technical solution adopted in this example and the corresponding results were as follows.
(1) Resting or anti-CD3/CD28 bead-activated CD8⁺ T cells (1 × 10⁴ cells) were lysed and Kyn levels were analyzed by ELISA. The analysis results were as shown in Fig. 4A.
(2) Activated CD8⁺ T cells were treated with PBS or Kyn (200 µM) for 24 h. The expression of PAT4 and SLC7A8 was determined by real-time PCR (left) and immunostaining (right). The determination results were as shown in Fig. 4B. Bar, 10 µM.
(3) Scramble shRNA or shRNA against SLC7A8 and PAT4 were delivered via retrovirus to activated CD8⁺ T cells for 24 h, and then the transduced T cells were treated with Kyn (200 µM) for 48 h. Kyn levels in the supernatants were determined by ELISA and the Kyn consumption was calculated. The determination results were as shown in Fig. 4C.
(4) The experimental steps were the same as those of step (3), except that the expression of PD-1 was analyzed by flow cytometry. The analysis results were as shown in Fig. 4D.
(5) Activated OT-1 T cells were transfected with scramble shRNA, PAT4 shRNAs (Sh1 and Sh2) or SLC7A8 shRNAs (Sh1 and Sh2) for 72 h, and then co-cutured with OVA-B16 TRCs. TRC apoptosis was analyzed by flow cytometry. The analysis results were as shown in Fig. 4E.
(6) The mRNA expression of PAT4 and SLC7A8 was determined in activated CD8⁺ T cells treated with PBS, Kyn, Kyn/CH-223191 (10 µM) or Kyn/DMF (20 µM) for 48 h. The determination results were as shown in Fig. 4F.
(7) Resting or activated CD8⁺ T cells were treated with PBS or Kyn for 48 h. Chip-qPCR analysis was performed with an anti-AhR antibody and qPCR primers specific for the SLC7A8 or PAT4 promotor. The analysis results were as shown in Fig. 4G.
(8) HEK293T cells were transiently co-transfected with AhR vector and the luciferase reporter PGL3 coupled to the SLC7A8 or PAT4 promotor, and then treated with Kyn for another 48 h. The determination results were as shown in Fig. 4H. ^{∗∗}p<0.01. The data represented mean ± SEM.

As could be seen from the results of Example 4, the inventors went on to investigate how exogenous Kyn entered T cells. Endogenous Kyn was barely detectable in CD8⁺ T cells (Fig. 4A), and these T cells were found to express very low levels of IDOl, suggesting that CD8⁺ T cells relied on exogenous Kyn to activate AhR so as to upregulate PD-1 expression. Several transporters including SLC1A5, SLC7A5, SLC7A8 and PAT4 which transported Kyn into cells were tested. Although these four transporters were found to be expressed in CD8⁺ T cells, the addition of Kyn strikingly resulted in the upregulation of the expression of SLC7A8 and PAT4 (Fig. 4B). Notably, compared to resting CD8⁺ T cells, the expression of SLC7A8 and PAT4 was upregulated in anti-CD3/CD28 bead-activated CD8⁺ T cells and further enhanced by Kyn treatment. In addition, the inventors found that IFN-γ treatment did not affect the expression of PAT4 and SLC7A8 in CD8⁺ T cells. These results prompted the inventors to speculate that these two transporters were the main Kyn import in CD8⁺ T cells. Therefore, the inventors used retrovirus to deliver SLC7A8 and PAT4 shRNAs into primary T cells or treated T cells with the inhibitor 2-amino-2-norbornanecarboxylic acid (BCH) against SLC7A8 or sarcosine against PAT4. The result showed that inhibition or knockdown of SLC7A8 and PAT4 blocked the entry of Kyn into T cells (Fig. 4C), inhibited AhR activity, abolished PD-1 upregulation (Fig. 4D), and enhanced OT-1 T cell cytotoxicity to OVA-B16 TRCs (Fig. 4E). Meanwhile, the inventors found that the AhR inhibitor DMF or CH-223191 was capable of effectively inhibiting Kyn-induced upregulation of SLC7A8 and PAT4 (Fig. 4F). Chip-PCR assay further confirmed that AhR indeed bound to the promoters of SLC7A8 and PAT4 genes (Fig. 4G). The luciferase assay also proved that the expression of SLC7A8 and PAT4 was boosted upon AhR binding (Fig. 4H). Regarding the antiport function in many transport systems, the inventors additionally analyzed the influence of SLC7A8 and PAT4 on the transport of other essential amino acids. After 48-hour treatment with or without Kyn, anti-CD3/CD28 bead-activated CD8⁺ T cells were used to analyze essential amino acids in the cytosol by LC-QE-MS. The result showed that Kyn treatment had no effect on most of the essential amino acids except some decrease of the intensity of threonine. Together, these data suggested that AhR transcriptionally activated Kyn transporters and promoted T cells to take up exogenous Kyn.

### Example 5: Kyn-AhR-regulated PD-1 pathway was independent of but could be facilitated by TCR signaling

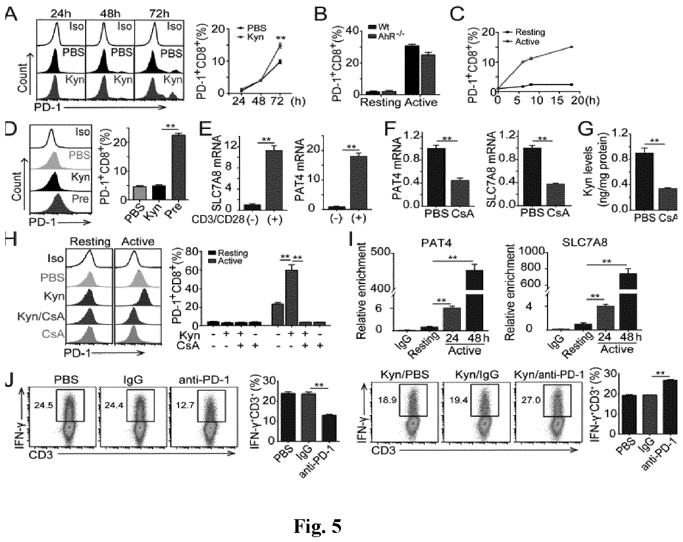

The steps of the technical solution adopted in this example and the corresponding results were as follows.
(1) CD8⁺ T cells were treated with Kyn (200 µM) for the time periods. The expression of PD-1 was determined by flow cytometry. The determination results were as shown in Fig. 5A.
(2) PD-1 expression was determined by flow cytometry in resting or anti-CD3/CD28 bead-activated CD8⁺ T cells isolated from wild type or AhR^{-/-} mice. The determination results were as shown in Fig. 5B.
(3) PD-1 expression in resting and CD3/CD28 bead-activated CD8⁺ T cells was determined by flow cytometry at the time points as shown in Fig. 5C. The determination results were as shown in Fig. 5C.
(4) CD8⁺ T cells were pretreated with anti-CD3/CD28 beads for 4 h, and then treated with PBS or Kyn for 24 h. PD-1 expression was determined by flow cytometry. The determination results were as shown in Fig. 5D.
(5) The mRNA level of SLC7A8 or PAT4 in resting or anti-CD3/CD28 bead-activated CD8⁺ T cells was determined. The determination results were as shown in Fig. 5E.
(6) Anti-CD3/CD28 bead-activated CD8⁺ T cells were treated with CsA (1 µM) for 48 h. The mRNA expression of PAT4 and SLC7A8 was determined by real-time PCR. The determination results were as shown in Fig. 5F.
(7) Activated CD8⁺ T cells were treated with Kyn (200 µM) in the presence or absence of CsA (1 µM) for 48 h. The cells were lysed and the Kyn level was determined by ELISA assay. The determination results were as shown in Fig. 5G.
(8) Resting or activated CD8⁺ T cells were treated with PBS, Kyn (200 µM), CsA (1 µM), Kyn/CsA for 48 h. The PD-1 level was determined by flow cytometry. The determination results were as shown in Fig. 5H.
(9) Chip-qPCR analysis was performed in resting or activated CD8⁺ T cells with an anti-NFATc1 antibody and qPCR primers specific for the PAT4 or SLC7A8 promotor. The analysis results were as shown in Fig. 51.
(10) Splenic APCs pulsed with OVA₂₅₇₋₂₆₄ (2 µg/ml) were co-cultured with OT-1 CD8⁺ T cells in the presence of IgG or anti-PD-1 neutralizing antibody (10 µg/ml) for 72 h. IFN-γ expression was measured by flow cytometry (left). Or, CD8⁺ T cells from OT-1 mice were treated with Kyn (200 µM) for 72 h, then co-cultured with OVA-peptide loaded APCs and treated with or without anti-PD-1 neutralizing antibody for 72 h (right). The determination results were as shown in Fig. J. ^{∗∗}p<0.01. The data represented mean ± SEM.

As could be seen from the results of Example 5, the inventors found that the Kyn-AhR pathway and TCR signaling could induce PD-1 expression independent of each other. Despite the aforementioned PD-1 upregulation by Kyn in the presence of T cell activation signals (Figs. 2D and 2E), Kyn alone was actually enough to upregulate PD-1 in resting CD8⁺ T cells (Fig. 5A). However, such induction was a slow process with an initial increase at the time point of 48 hours. On the other hand, CD8⁺ T cells with AhR deficiency also upregulated the expression of PD-1 upon T cell activation signaling (Fig. 5B). Notably, the inventors found that TCR signaling-induced PD-1 expression was a rapid process with an initial increase at the time point of initial hours (Fig. 5C). The inventors incubated CD8⁺ T cells with anti-CD3/CD28 microbeads for 4 hours. After the removal of the beads, these cells were treated with Kyn. The inventors found that this short TCR-activation resulted in higher PD-1 expression (Fig. 5D), suggesting that TCR signaling facilitated PD-1 induction by Kyn-AhR. Since Kyn importation was the limiting step in the Kyn-AhR dependent pathway, the inventors investigated whether and how TCR signaling affected Kyn-transporters. The inventors found that naive T cells expressed low levels of SLC7A8 and PAT4, while activated T cells had significantly higher expression of the two transporters (Fig. 5E). During CD8⁺ T cell activation, a wide array of TCR signaling-triggered transcription factors were activated and might be involved in enhancing PD-1 expression. Among them, nuclear factor of activated T cells c1 (NFATc1) appeared to be critical. NFATc1 was normally localized in the cytosol as a phosphorylated inactive form but translocated into the nucleus upon dephosphorylation. The inhibitor cyclosporine A (CsA) blocked NFATc1 nuclear translocation and inhibited the expression of PD-1 in CD8⁺ T cells. When the inventors used CsA to block NFATc1, the inventors found that the upregulation of SLC7A8 and PAT4 in activated CD8⁺ T cells was also blocked (Fig. 5F), and consistent with this, the inventors also observed diminished uptake of Kyn (Fig. 5G). The promotive effect of TCR signaling on Kyn/AhR-induced PD-1 expression was also inhibited by CsA (Fig. 5H). Moreover, an analysis using the Jaspar and Genomatrix softwares revealed the presence of multiple consensus cis-elements for NFATc1 binding on the promoters of SLC7A8 and PAT4, which was confirmed by the ChIP assay showing that NFATc1 indeed bound to the promoters (Fig. 51). By co-culturing CD8⁺ T cells with antigen presenting cells (APCs), the inventors found that PD-1 blockade inhibited the production of IFN-γ while Kyn treatment upregulated the production of IFN-γ upon PD-1 blockade (Fig. 5J). Together, these data suggested that Kyn-AhR-regulated PD-1 pathway was independent of but could be facilitated by TCR signaling.

### Example 6: Kyn/AhR regulated PD-1 expression of CD8⁺ T cells in tumor-bearing mice

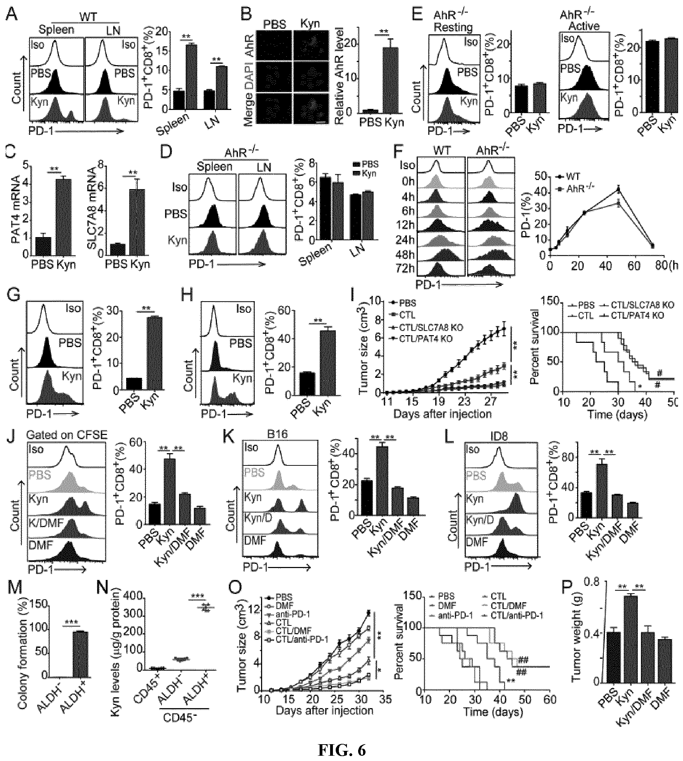

The steps of the technical solution adopted in this example and the corresponding results were as follows.
(1) WT C57BL/6 mice were i.p. injected with PBS or Kyn (50 µg, once per day) for 5 days, and CD8⁺ T cells were sorted by FACS from the spleen and mesenteric lymph nodes. PD-1 expression was determined by flow cytometry (Fig. 6A). Some CD8⁺ T cells from spleen were immunostained with AhR and DAPI (Fig. 6B), and the mRNA expression of PAT4 and SLC7A8 was determined by real-time PCR (Fig. 6C). The above experimental results were as shown in Fig. 6A, Fig. 6B and Fig. 6C, respectively. Bar, 10 µM.
(2) The experimental steps were the same as those of step (1), except that AhR^{-/-} mice were used. The experimental results were as shown in Fig. 6D.
(3) PD-1 expression in resting or anti-CD3/CD28 bead-activated splenic CD8⁺ T cells was determined, wherein said cells were isolated from AhR^{-/-} C57BL/6 mice injected with PBS or Kyn for 5 days. The determination results of the expression were as shown in Fig. 6E.
(4) WT or AhR^{-/-} C57BL/6 mice were injected with or without anti-CD3 antibody (5 µg, i.p.) for different time periods and PD-1 expression was determined by flow cytometry. The determination results were as shown in Fig. 6F.
(5) C57BL/6 mice were injected with anti-CD3 antibody for 72 h, and then these mice were given Kyn (50 µg). The PD-1 expression in splenic CD8⁺ T cells was determined by flow cytometry after 48 h. The determination results were as shown in Fig. 6G.
(6) C57BL/6 mice with 5 × 5 mm OVA-B16 melanoma were adoptively transferred with CFSE-labeled OT-1 CD8⁺ T cells (4 × 10⁶ cells/mouse, once every three days) on three occasions. At the same time, some mice were i.p. injected with Kyn (50 µg/mouse, once every two days) for three times. On Day 7 after transferring, CFSE⁺CD8⁺ T cells were isolated from tumor for flow cytometric analysis of PD-1 expression. The analysis results were as shown in Fig. 6H.
(7) 4 × 10⁶ CD45.2 OT-1 T cells transfected with scramble-, shPAT4- or shSLC7A8-retrovirus were adoptively transferred into CD45.1 mice with 5 × 5 mm OVA-B16 melanoma for three times (once every three days). The tumor growth was measured (left) and the long-term survival rate was analyzed (right). The measurement results were as shown in Fig. 61.
(8) The experimental steps were the same as those of step (7), except that C57BL/6 mice were treated with Kyn, DMF (5 µg/mouse, o.t., twice every day) or Kyn + DMF for 6 days. CFSE⁺CD8⁺ T cells from tumor were isolated for flow cytometric analysis of PD-1 expression. The analysis results were as shown in Fig. 6J.
(9) C57BL/6 mice with 5 × 5 mm B16 melanoma were treated with PBS, Kyn (50 µg/mouse, i.p., once every two days), DMF (5 µg/mouse, o.t., once every day) or Kyn + DMF for 6 days. PD-1 expression in CD8⁺ T cells isolated from tumors was determined by flow cytometry (n=5). The determination results were as shown in Fig. 6K.
(10) C57BL/6 mice were intraperitoneally injected with 1 × 10⁶ ID8 ovarian cancer cells. After 10 days, mice were intraperitoneally administered with PBS, Kyn, DMF or Kyn + DMF for 6 days. CD8⁺ T cells from ascites were isolated and PD-1 expression was determined by flow cytometry (n=5). The determination results were as shown in Fig. 6L.
(11) CD45⁻ALDH⁺ and CD45⁻ALDH⁻ tumor cells were sorted from spontaneously formed breast tumor in MMTV-PyMT mice. The cells were seeded into 90 Pa soft 3D fibrin gels, respectively. After 4 days, the number of colonies was counted. The counting results were as shown in Fig. 6M.
(12) CD45⁺, CD45⁻ALDH⁺ and CD45⁻ALDH⁻ cells were lysed and the Kyn levels were determined by ELISA. The determination results were as shown in Fig. 6N.
(13) C57BL/6 mice with 5 × 5 mm OVA-B16 melanoma were adoptively transferred with or without OT-1 CD8⁺ T cells (4 × 10⁶ cells/mouse) once per three days for three times. At the same time, these mice were treated with PBS, DMF (5 µg/mouse, o.t., twice every day) or anti-PD-1 neutralizing antibody (250 µg/day, once every two days, twice) for 20 days. The tumor growth was measured (left) and the long-term survival rate was analyzed (right). The measurement results were as shown in Fig. 6O.
(14) The experimental steps were the same as those of step (9), except that the tumors were weighed. The weighing results were as shown in Fig. 6P. ^{∗}p < 0.05, ^{∗∗}p < 0.01; #p < 0.05, ##p < 0.01, as compared with CTL group. The data represented mean ± SEM.

As could be seen from the results of Example 6, the analysis of spleen and lymph nodes showed that CD8⁺ T cells did not undergo proliferation but upregulated PD-1 expression on Day 5 (Fig. 6A). Meanwhile, the inventors observed that AhR in these CD8⁺ T cells was translocated to the nucleus (Fig. 6B), and that the Kyn transporters SLC7A8 and PAT4 were upregulated (Fig. 6C). In agreement with the above *in vitro* data, such exogenous Kyn failed to induce the PD-1 expression in AhR^{-/-} mice (Figs. 6D and 6E). On the other hand, injection of stimulatory anti-CD3 antibody into WT or AhR^{-/-} mice rapidly upregulated PD-1 in splenic CD8⁺ T cells as observed at the 6-hour time point, but PD-1 level started to decline after 72 hours (Fig. 6F). At this 72-hour time point, the inventors injected the mice with Kyn. The inventors found that the Kyn-induced PD-1 expression was enhanced by the first TCR signaling (Fig. 6G), further suggesting that TCR signaling facilitated PD-1 induction by Kyn-AhR. In order to study the upregulation of PD-1 in an *in-vivo* tumor model, the inventors adoptively transferred CD45.2 OT-1 T cells into CD45.1 mice three days after subcutaneous injection of OVA-B16 TRCs (5 × 10⁵/mouse). At different time points, the inventors analyzed PD-1 expression and measured Kyn concentration at tumor sites. The inventors found that Kyn levels were gradually increased and reached a plateau on Day 9. Coincidently, PD-1 expression in CD45.2 OT-1 T cells was also gradually elevated and reached a plateau on Day 9, implying an intrinsic correlation between Kyn and PD-1 expression. Afterwards, the inventors treated OVA-B16 melanoma (5 × 5 mm)-bearing mice with Kyn after the adoptive transfer of CFSE-labeled OT-1 T cells. Consistent with the above-mentioned data, the inventors observed much higher levels of PD-1 expression in tumor-infiltrating CFSE⁺ T cells (Fig. 6H).

Next, the inventors adoptively transferred scramble, shPAT4 or shSLC7A8 OT-1 T cells into CD45.1 mice bearing 3 × 3 mm OVA-B16 melanoma. The inventors found that PAT4 or SLC7A8 knockdown remarkably decreased PD-1 expression but increased the expression of IFN-γ and TNF-α in OT-1 T cells, concomitant with retarded tumor growth as well as prolonged survival of the mice (Fig. 6I), suggesting that Kyn triggered PD-1 expression in tumor microenvironment. Also, this Kyn-triggered upregulation of PD-1 expression in tumor-specific CD8⁺ T cells was abrogated in the presence of AhR-inhibitor DMF, as observed among CFSE⁺ T cells isolated from the tumor mass, the spleen or the lymph nodes (Fig. 6J). Consistently, upregulation of the expression of IFN-γ and TNF-α in T cells was observed. In addition, B16 melanoma-bearing mice also received the treatment with Kyn, Kyn + DMF or DMF. Consistently, the inventors found that Kyn could upregulate PD-1 expression in CD8⁺ T cells isolated from tumor, spleen or lymph nodes, which, however, was blocked by DMF (Fig. 6K). Peritoneal neoplasia including ovarian cancer was infiltrated with CD8⁺ T cells that commonly upregulated PD-1 expression. Here, the inventors additionally used ID8 ovarian cancer and H22 hepatocarcinoma ascites, two murine peritoneal tumor models, to verify the above PD-1 expression results. Similarly, PD-1 expression in peritoneal, splenic or lymph node CD8⁺ T cells was promoted by Kyn and the expression of IFN-γ and TNF-α was inhibited (Fig. 6L), while the expression pattern of PD-1, IFN-γ and TNF-α was reversed by DMF.

In spontaneously formed breast cancer MMTV-PyMT mice, CD4S⁻ALDH⁺ tumor cells had been identified as tumorigenic cells. The inventors further identified them as TRCs, since only CD4S⁻ALDH⁺ but not CD45⁻ALDH⁻ tumor cells grew into colonies in 90 Pa 3D soft fibrin gels (Fig. 6M). As expected, IDO1 expression and Kyn levels were very low in CD45⁻ALDH⁻tumor cells as well as in CD45⁺ immune cells, but remarkably high in CD4S⁻ALDH⁺ TRCs (Fig. 6N). Consistently, supernatants from IFN-γ-treated CD45⁻ALDH⁺ but not CD45⁻ALDH⁻ cells upregulated PD-1 expression in CD8⁺ T cells. On the other hand, the inventors intratumorally injected same amount of OVA-B16 TRCs or OVA-B16 cells into melanoma-bearing CD45.1 C57BL/6 mice, concomitant with the adoptive transfer of CD45.2 OT-1 cells. The inventors found that OT-1 T cells in the OVA-B16 TRC group expressed much higher PD-1 than that in the OVA-B16 group 3 days later. Meanwhile, IDO1 knockdown in B16 TRC-formed melanoma resulted in a significant decrease of PD-1 expression in adoptively transferred gp-100 specific CD8⁺ T cells as well as the increased expression of IFN-γ and TNF-α in these T cells.

The inventors co-cultured OVA-B16 TRCs, B16 TRCs, OVA-B16 cells or B16 cells with OVA-specific CD45.2 OT-1 T cells and OVA-nonspecific CD45.1 CD8⁺ T cells for 24 hours. The inventors found that PD-1 upregulation in non-specific CD45.1 CD8⁺ T cells was not induced by the above tumor cell types; PD-1 upregulation in antigen-specific OT-1 T cells was only slightly induced by OVA-B16 cells; however, PD-1 upregulation in OT-1 T cells was strikingly induced by OVA-B16 TRCs, suggesting that PD-1 expression was not only antigen-restricted but also controlled by local Kyn concentration. The adoptive transfer of OVA-specific CD8⁺ T cells generated the therapeutic effect against OVA-B16 melanoma in mice, which was enhanced by the administration of PD-1 antibody (Fig. 6O). Comparable to PD-1 antibody, use of DMF also led to equal treatment efficiency (Fig. 6O). In addition, the accelerated tumor growth seen with Kyn-treatment was also reversed by DMF-treatment (Fig. 6P). Together, these data suggested that PD-1 expression of CD8⁺ T cells in tumor-bearing mice was regulated through the Kyn-AhR pathway.

### Example 7: Upregulation of PD-1 expression in CD8⁺ T cells in cancer patients by Kyn-AhR pathway

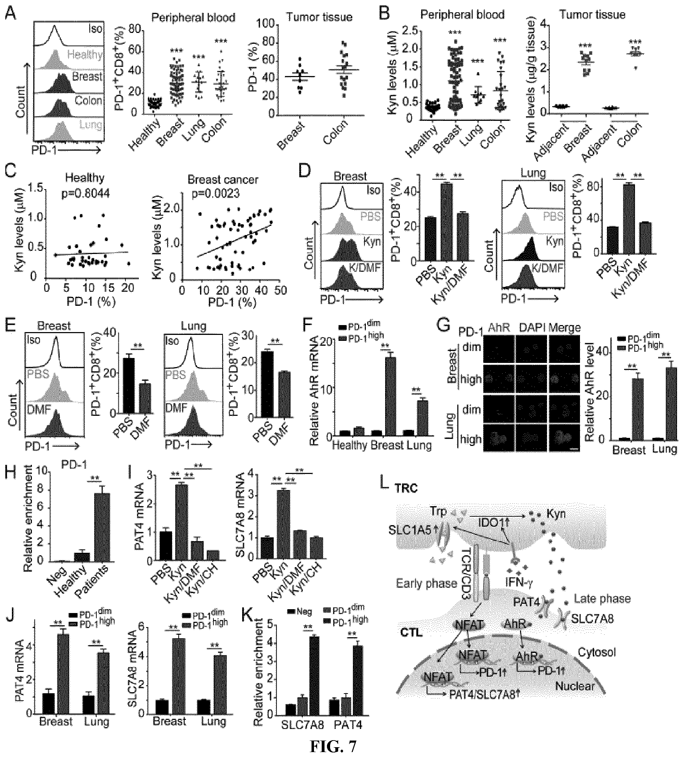

The steps of the technical solution adopted in this example and the corresponding results were as follows.
(1) CD8⁺ T cells were isolated from the peripheral blood of healthy volunteers (n = 50) or patients with breast cancer (n = 84), colon cancer (n = 28) or lung cancer (n = 15). Or, CD8⁺ T cells were isolated from breast tumor tissues (n = 9) or colon tumor tissues (n = 17). PD-1 expression was analyzed by flow cytometry. The analysis results were as shown in Fig. 7.
(2) Kyn levels in the serum of healthy volunteers (n = 31) and patients with breast cancer (n = 73), lung cancer (n = 10) or colon cancer (n = 26) were measured. Or, lysates from fresh human breast tumor tissues (n = 11) or colon tumor tissues (n = 8) and the adjacent normal tissues were prepared and Kyn levels were determined. The determination results were as shown in Fig. 7B.
(3) The correlation between PD-1 expression and Kyn level in healthy people (left, n = 37) and patients with breast cancer (right, n = 62) was analyzed. The analysis results were as shown in Fig. 7C.
(4) CD8⁺ T cells isolated from PBMCs of patients with breast cancer or lung cancer were activated by anti-CD3/CD28 beads and treated with PBS, Kyn (200 µM) or Kyn/DMF (20 µM) for 48 h. The expression of PD-1 was determined by flow cytometry. The determination results were as shown in Fig. 7D.
(5) CD8⁺ T cells isolated from patients with breast cancer or lung cancer were treated with DMF (20 µM) for 48 h. PD-1 expression was determined by flow cytometry (n=6). The determination results were as shown in Fig. 7E.
(6) PD-1^{hlgh} CD8⁺ or PD-1^{dim} CD8⁺ T cells were sorted from PBMCs of healthy donors or patients with breast cancer or lung cancer. The mRNA expression of AhR was analyzed by real-time PCR (n = 8, Fig. 7F). These cell populations were also immunostained with anti-AhR antibody and DAPI and imaged by confocal microscopy (Fig. 7G). The experimental results were as shown in Fig. 7F and Fig. 7G, respectively. Bar, 10 µM.
(7) Chip-qPCR analysis of AhR binding to the PD-1 promotor was performed. The analysis results were as shown in Fig. 7H.
(8) CD8⁺ T cells from breast cancer patients were treated with PBS, Kyn (200 µM), Kyn/DMF (20 µM) or Kyn/CH (50 µM) for 48 h. The expression of PAT4 and SLC7A8 was determined by real-time PCR. The determination results were as shown in Fig. 71.
(9) The mRNA expression of PAT4 or SLC7A8 in PD-1^{hlgh} CD8⁺ T cells or PD-1^{dim} CD8⁺ T cells from breast cancer patients and lung cancer patients was determined. The results of expression were as shown in Fig. 7J.
(10) Chip-qPCR analysis was performed in PD-1^{high} CD8⁺ T cells or PD-1^{dim} CD8⁺ T cells isolated from the PBMCs of breast cancer patients with an anti-AhR antibody and qPCR primers specific for the promotor of SLC7A8 or PAT4. The analysis results were as shown in Fig. 7K. The data represented mean ± SEM.
(11) How PD-1 was upregulated in CD8⁺ T cells was shown in the schematic diagram. The results were as shown in Fig. 7L. ^{∗∗}p<0.01, ^{∗∗∗}p<0.001. The data represented mean ± SEM.

As could be seen from the results of Example 7, flow cytometric analysis showed that peripheral blood CD8⁺ T cells from breast, colon and lung cancer patients had higher PD-1 expression, compared to healthy donor controls (Fig. 7A). Moreover, very high PD-1 expression was found in tumor-infiltrating CD8⁺ T cells of breast and colon cancer patients (Fig. 7A). In line with these results, Kyn levels in plasma and tumor tissues were strikingly elevated in these cancer patients (Fig. 7B). It was worth noting that Kyn levels were strongly correlated with PD-1 expression in breast and colon cancer patients, but not in healthy people (Fig. 7C). To clarify the cellular source of the Kyn production, CD45⁺ immune cells and CD45⁻ tumor cells isolated from breast tumor tissues (n = 10) and colon tumor tissues (n = 14) of patients were analyzed. IDO1 expression as well as Kyn levels were dominantly high in CD45⁻ cells rather than CD45⁺ cells. Then, the inventors treated CD45⁻ tumor cells with IFN-γ in the presence or absence of 1-MT for 24 hours *in vitro.* The supernatants were used to treat peripheral blood T cells of patients for 24 hours. Flow cytometric analysis showed that PD-1 expression was upregulated in CD8⁺ T cells, while this process was blocked by the addition of 1-MT, suggesting that Kyn in human tumor tissues was mainly produced by tumor cells, which promoted the expression of PD-1 in CD8⁺ T cells. Considering that Kyn levels were regulated by Trp transporter, the inventors additionally found that high levels of SLC1A5 were expressed in breast and colon tumor tissues of patients.

The inventors used Kyn to treat the above CD8⁺ T cells from human samples *in vitro* with or without the stimulation of anti-CD3/CD28 microbeads. Kyn upregulated PD-1 expression in both resting and activated CD8⁺ T cells from healthy donors as well as breast and lung cancer patients (Fig. 7D). Furthermore, Kyn treatment promoted the translocation of AhR from the cytosol to nucleus in both resting and activated peripheral CD8⁺ T cells from breast and lung cancer patients. Consistently, AhR nuclear translocation was also found in tumor-infiltrating T cells of breast and colon cancer patients. The inventors used DMF or CH-223191 to block AhR activity. The inventors found that the addition of DMF or CH-223191 inhibited Kyn-induced PD-1 upregulation in human CD8⁺ T cells (Fig. 7D). More significantly, the addition of DMF alone could directly downregulate PD-1 expression in patients' CD8⁺ T cells (Fig. 7E), suggesting that AhR might be a critical signal molecule that drove PD-1 expression in CD8⁺ T cells of cancer patients.

The inventors compared the AhR activity between PD-1^{high} and PD-l^{dim} CD8⁺ T cells of cancer patients. The inventors found that PD-1^{high}CD8⁺ T cells, sorted from the lung and breast cancer patients but not from healthy controls, expressed much higher levels of AhR mRNA than PD-1^{dim}CD8⁺ T cells from the corresponding settings (Fig. 7F). Further immunostaining analysis confirmed that PD-1^{high} T cells from patients indeed had more AhR activity, as evidenced by nuclear translocation (Fig. 7G), and such nuclear AhR strongly bound to the promoter of PD-1 in CD8⁺ T cells from breast cancer patients compared to the healthy control (Fig. 7H). SLC7A8 and PAT4 were involved in PD-1 expression via transporting exogenous Kyn and were regulated by AhR in murine CD8⁺ T cells (Figs. 4D and 4E).

SLC7A8 and PAT4 were upregulated in human CD8⁺ T cells upon the addition of Kyn (Fig. 71). Immunohistochemical staining showed that PAT4 was highly expressed in tumor-infiltrating T cells of breast and colon cancer patients. However, such upregulation could be blocked by the AhR inhibitor DMF or CH-223191 (Fig. 71). Consistently, only PD-1^{hlgh} but not PD-1^{dim} CD8⁺ T cells sorted from cancer patients had high expression of SLC7A5 and PAT4 (Fig. 7J). Moreover, the binding of AhR to the promoters of SLC7A8 and PAT4 and the regulation of their expression were confirmed in human CD8⁺ T cells (Fig. 7K). Together, these data suggested that the elevated PD-1 expression in CD8⁺ T cells of cancer patients might be mainly driven by the Kyn-AhR pathway.

### Example 8: GPNA (L-γ-Glutamyl-p-nitroanilide) was capable of significantly inhibiting the uptake of tryptophan by tumor cells and reducing PD-1 expression in specific OT-1 CD8⁺ T cells

### 1. Experiment to determine the effect of GPNA on the uptake of tryptophan by tumor cells

### 1) Experimental steps

Mouse OVA-B16 cells were cultured in RPMI-1640 culture medium *in vitro* and were group and treated as follows.
Control group 1 (PBS): culture medium + PBS;
Control group 2 (GPNA): culture medium + 10 µM GPNA;
Experimental group 3 (IFN-γ): culture medium + 50 ng/mL IFN-γ;
Experimental group 4 (IFN-γ + GPNA): culture medium + 50 ng/mL IFN-γ + 10 µM GPNA;

The cells in each group were collected after 24 h and ultrasonically broken, the concentrations of tryptophan in the lysates were determined by ELISA, and BCA protein assay was carried out at the same time.

### 2) Experimental results

As could be seen from Fig. 8A, IFN-γ was capable of significantly increasing the uptake of tryptophan by tumor cells, and the uptake of tryptophan by tumor cells was significantly reduced after using GPNA to inhibit the tryptophan transporter SLC1A5.

### 2. Experiment to determine the effect of GPNA on PD-1 expression in specific CD8⁺ T cells

### 1) Experimental steps

3D-cultured OVA-B16 cells were grouped and treated with drug as follows.
Control group 1 (PBS): culture medium + PBS;
Experimental group 2 (GPNA): culture medium + 10 µM GPNA;
After 12 h of treatment, the cells were co-cultured with OT-1 CD8⁺ T cells that had been activated for 48 h at a ratio of 1:10, and PD-1 expression in CD8⁺ T cells was determined by flow cytometry after 4 h.

### 2) Experimental results

As could be seen from Fig. 8B, GPNA inhibited the uptake of tryptophan by tumor cells and could significantly inhibit PD-1 expression in specific OT-1 CD8⁺ T cells co-cultured with tumor cells.

### Example 9: SAR (sarcosine) and BCH (2-amino-2-norbornanecarboxylic acid) were capable of significantly inhibiting PD-1 expression in CD8⁺ T cells

### 1. Experiment to determine the effect of Kyn on the expression of PAT4 and SLC7A8 in CD8⁺ T cells

### 1) Experimental steps

Splenic CD8⁺ T cells of OT-1 mice were obtained by magnetic bead sorting, and seeded into a 24-well plate (1 million cells/well) while anti-CD3/CD28 magnetic beads were added to activate T cells. The culture medium was RPMI-1640 added with 10 ng/mL mouse IL-2 and 50 nM β-mercaptoethanol. Cells were grouped and treated with drugs as follows.
Control group 1 (PBS): culture medium + PBS;
Experimental group 2 (Kyn): culture medium + 200 µM Kyn;
RNA was collected from cells in each group after 48 h, and the expression of PAT4 and SLC7A8 was determined by fluorescence quantitative PCR.

### 2) Experimental results

As could be seen from Fig. 9A, Kyn was capable of significantly upregulating PAT4 and SLC7A8 in mouse CD8⁺ T cells, indicating that the transporters PAT4 and SLC7A8 played an important role in the transport process of Kyn.

### 2. Experiment to determine the effect of SAR (sarcosine) and BCH on PD-1 expression in CD8⁺ T cells

### 1) Experimental steps

Mouse splenic CD8⁺ T cells were obtained by magnetic bead sorting, and seeded into a U-shaped bottom 96-well plate (100000 cells/well) while anti-CD3/CD28 magnetic beads were added to activate T cells. The culture medium was RPMI-1640 added with 10 ng/mL mouse IL-2 and 50nM β-mercaptoethanol. Cells were grouped and treated with drugs as follows.
Control group 1 (PBS): culture medium + PBS;
Experimental group 2 (Kyn): culture medium + 200 µM Kyn;
Experimental group 3 (Kyn + SAR): culture medium + 200 µM Kyn + 10 mM SAR;
Experimental group 4 (Kyn + BCH): culture medium + 200 µM Kyn + 100 µM BCH;
The expression level of PD-1 in CD8⁺ T cells of each group was determined by flow cytometry after 48 h.

### 2) Experimental results

As could be seen from Fig. 9B, based on the fact that the expression of PD-1 in CD8⁺ T cells was significant increased by Kyn, using SAR and BCH to respectively inhibit the Kyn transporters PAT4 and SLC7A8 was capable of significantly reducing the expression of PD-1 in CD8⁺ T cells.

### 3. Experiment to determine the effects of SAR and BCH on the function of specific CD8⁺ T cells

### 1) Experimental steps

Splenic CD8⁺ T cells of OT-1 mouse were obtained by magnetic bead sorting and seeded into a U-shaped bottom 96-well plate (100000 cells/well). Cells were grouped and treated with drugs as follows.
Control group 1 (PBS): culture medium + PBS;
Experimental group 2 (SAR): culture medium + 10 mM SAR;
Experimental group 3 (BCH): culture medium + 100 µM BCH;
After T cells were pretreated for 24 hours, 3D-cultured OVA-B16 cells were co-cultured with OT-1 CD8⁺ T cells at a ratio of 1:10, and the apoptosis of OVA-B16 cells was determined by flow cytometry after 4 hours.

### 2) Experimental results

As could be seen from Fig. 9C, the apoptosis ratio of OVA-B16 tumor cells that were co-cultured with T cells pretreated with SAR and BCH was increased significantly, indicating that SAR and BCH were capable of significantly inhibiting the uptake of Kyn by T cells, downregulating PD-1 expression and enhancing the killing effect of CD8⁺ T cells.

### Example 10: SAR and BCH were capable of significantly inhibiting PD-1 expression in human peripheral blood CD8⁺ T cells

### 1. Experiment to determine the effect of Kyn on the expression of PAT4 and SLC7A8 in human peripheral blood CD8⁺ T cells

### 1) Experimental steps

Mononuclear cells were isolated and obtained from the peripheral whole blood of breast cancer patients by using Ficoll. CD8⁺ T cells were sorted by magnetic beads, and seeded into a U-shaped bottom 96-well plate (100000 cells/well) while anti-CD3/CD28 magnetic beads were added to activate T cells. The culture medium was RPMI-1640 added with 10 ng/mL human IL-2. Cells were grouped and treated with drugs as follows.
Control group 1 (PBS): culture medium + PBS;
Experimental group 2 (Kyn): culture medium + 200 µM Kyn;
RNA was collected from cells in each group after 48 h, and the expression of PAT4 and SLC7A8 was determined by fluorescence quantitative PCR.

### 2) Experimental results

As could be seen from Fig. 10A, Kyn was capable of significantly upregulating PAT4 and SLC7A8 in peripheral blood CD8⁺ T cells of breast cancer patients, indicating that the transporters PAT4 and SLC7A8 played an important role in the transport process of Kyn.

### 2. Experiment to determine the effects of SAR and BCH on PD-1 expression in human peripheral blood CD8⁺ T cells

### 1) Experimental steps

Mononuclear cells were isolated and obtained from the peripheral whole blood of breast cancer patients by using Ficoll. CD8⁺ T cells were sorted by magnetic beads, and seeded into a U-shaped bottom 96-well plate (100000 cells/well) while anti-CD3/CD28 magnetic beads were added to activate T cells. The culture medium was RPMI-1640 added with 10 ng/mL human IL-2. Cells were grouped and treated with drugs as follows. Control group 1 (PBS): culture medium + PBS;
Experimental group 2 (Kyn): culture medium + 200 µM Kyn;
Experimental group 3 (Kyn + SAR): culture medium + 200 µM Kyn + 10 mM SAR;
Experimental group 4 (Kyn + BCH): culture medium + 200 µM Kyn + 100 µM BCH;
The expression level of PD-1 in CD8⁺ T cells of each group was determined by flow cytometry after 48 h.

### 2) Experimental results

As could be seen from Fig. 10B, based on the fact that PD-1 expression in peripheral blood CD8⁺ T cells of breast cancer patients was significant increased by Kyn, using SAR and BCH to respectively inhibit the Kyn transporters PAT4 and SLC7A8 was capable of significantly reducing the expression of PD-1 in CD8⁺ T cells.

According to the results of Examples 8 to 10 of the present disclosure, both using the inhibitor GPNA to inhibit the tryptophan transporter in tumor cells and using the inhibitors SAR and BCH to respectively inhibit the Kyn transporters PAT4 and SLC7A8 in CD8⁺ T cells could effectively reduce PD-1 expression in specific CD8⁺ T cells and enhance the killing effect of specific CD8⁺ T cells on tumor cells.

## Claims

1. A transport protein inhibitor selected from inhibitors of SLC1A5, SLC7A8 and PAT4 for use in preventing or treating cancer by reducing the expression of PD-1, wherein the patient has been identified as having cancer accompanied with increased PD-1 expression, and wherein the transport protein inhibitor is selected from one or more of L-γ-glutamyl-p-nitroanilide (GPNA), sarcosine (SAR), 2-amino-2-norboranecarboxylic acid (BCH), and pharmacologically acceptable salts thereof.

2. The transport protein inhibitor for the use according to claim 1, wherein the cancer accompanied with increased PD-1 expression is selected from one or more of melanoma, liver cancer, breast cancer, lung cancer, colorectal cancer, ovarian cancer, or leukemia.

3. The transport protein inhibitor for the use according to claim 1, in combination with other agent(s) for treating the cancer accompanied with increased PD-1 expression; preferably, said other agent for treating the cancer accompanied with increased PD-1 expression is selected from one or more of an immunomodulator, an indoleamine 2,3-dioxygenase (IDO) inhibitor, an aryl hydrocarbon receptor (AhR) inhibitor, a radiotherapeutic agent or a chemotherapeutic agent; alternatively, the immunomodulator is selected from an immune checkpoint modulator or an agent that facilitates or mediates an antigen presentation that promotes a cell-mediated immune response.

4. The transport protein inhibitor for the use according to claim 1, wherein treating or preventing the cancer accompanied with increased PD-1 expression further comprises a step of surgical treatment.

## Patentansprüche

1. Transportproteininhibitor, ausgewählt aus Inhibitoren von SLC1A5, SLC7A8 und PAT4, zur Verwendung bei der Vorbeugung oder Behandlung von Krebs durch Verringerung der Expression von PD-1, wobei bei dem Patienten Krebs identifiziert wurde, der mit einer erhöhten PD-1-Expression einhergeht, und wobei der Transportproteininhibitor ausgewählt ist aus einem oder mehreren von L-γ-Glutamyl-p-nitroanilid (GPNA), Sarkosin (SAR), 2-Amino-2-norbornancarbonsäure (BCH) und pharmakologisch annehmbaren Salzen davon.

2. Transportproteininhibitor zur Verwendung nach Anspruch 1, wobei der Krebs, der mit einer erhöhten PD-1-Expression einhergeht, aus Melanom, Leberkrebs, Brustkrebs, Lungenkrebs, Kolorektalkrebs, Eierstockkrebs und Leukämie oder Kombinationen davon ausgewählt ist.

3. Transportproteininhibitor zur Verwendung nach Anspruch 1 in Kombination mit einem oder mehreren anderen Mitteln zur Behandlung des Krebses, der mit einer erhöhten PD-1-Expression einhergeht, wobei das andere Mittel zur Behandlung des Krebses, der mit einer erhöhten PD-1-Expression einhergeht, vorzugsweise ausgewählt ist aus einem oder mehreren von einem Immunmodulator, einem Indolamin-2,3-Dioxygenase (IDO)-Inhibitor, einem Aryl-Kohlenwasserstoffrezeptor (AhR)-Inhibitor, einem radiotherapeutischen Mittel oder einem chemotherapeutischen Mittel; wobei der Immunmodulator alternativ ausgewählt ist aus einem Immun-Checkpoint-Modulator oder einem Mittel, das eine Antigenpräsentation erleichtert oder vermittelt, die eine zellvermittelte Immunantwort fördert.

4. Transportproteininhibitor zur Verwendung nach Anspruch 1, wobei die Behandlung oder Vorbeugung des Krebses, der mit einer erhöhten PD-1-Expression einhergeht, ferner einen Schritt der chirurgischen Behandlung umfasst.

## Revendications

1. Inhibiteur de protéine de transport sélectionné parmi les inhibiteurs de SLC1A5, SLC7A8 et PAT4 pour utilisation dans la prévention ou le traitement d'un cancer par réduction de l'expression de PD-1, dans laquelle le patient a été identifié comme ayant un cancer accompagné d'une expression accrue de PD-1, et dans laquelle l'inhibiteur de protéine de transport est sélectionné parmi un ou plusieurs du L-γ-glutamyl-p-nitroanilide (GPNA), de la sarcosine (SAR), de l'acide 2-amino-2-norboranecarboxylique (BCH), et des sels pharmaceutiquement acceptables de ceux-ci.

2. Inhibiteur de protéine de transport pour utilisation selon la revendication 1, dans laquelle le cancer accompagné d'une expression accrue de PD-1 est sélectionné parmi un ou plusieurs d'un mélanome, d'un cancer du foie, d'un cancer du sein, d'un cancer du poumon, d'un cancer colorectal, d'un cancer ovarien, ou d'une leucémie.

3. Inhibiteur de protéine de transport pour utilisation selon la revendication 1, en combinaison avec un autre (d'autres) agent(s) pour le traitement du cancer accompagné d'une expression accrue de PD-1 ; de préférence ledit autre agent pour le traitement du cancer accompagné d'une expression accrue de PD-1 est sélectionné parmi un ou plusieurs d'un immunomodulateur, d'un inhibiteur de l'indoleamine 2,3-dioxygénase (IDO), d'un inhibiteur des récepteurs aux aryl hydrocarbures (AhR), d'un agent radiothérapeutique ou d'un agent chimiothérapeutique ; en variante, l'immunomodulateur est sélectionné parmi un modulateur des points de contrôle immunitaire ou un agent qui facilite ou médie une présentation de l'antigène qui favorise une réponse immunitaire à médiation cellulaire.

4. Inhibiteur de protéine de transport pour utilisation selon la revendication 1, dans laquelle le traitement ou la prévention du cancer accompagné d'une expression accrue de PD-1 comprend en outre une étape de traitement chirurgical.
